(19) Europäisches Patentamt
European Patent Office
Office européen des brevets

(11) Veröffentlichungsnummer: **0 001 751**
**B1**

(12) # EUROPÄISCHE PATENTSCHRIFT

(21) Anmeldenummer: **78100999.8**

(22) Anmeldetag: **27.09.78**

(51) Int. Cl.³: **C 07 D 233/56,**
A 01 N 43/50,
C 07 D 233/68

(54) **N-(Imidazolylmethyl)-acetanilide, ihre Verwendung als Herbizide und Verfahren zur Bekämpfung unerwünschten Pflanzenwuchses**

(30) Priorität: **03.10.77 DE 2744396**

(43) Veröffentlichungstag der Anmeldung:
**16.05.79 Patentblatt 79/10**

(45) Bekanntmachung des Hinweises auf die Patenterteilung:
**17.09.80 Patentblatt 80/19**

(84) Benannte Vertragsstaaten:
**BE CH DE FR GB LU NL SE**

(56) Entgegenhaltungen:
**DE - A - 2 226 593**
**DE - A - 2 405 510**
**DE - A - 2 648 008**
**US - A - 3 547 620**

(73) Patentinhaber: **BASF Aktiengesellschaft**
**Carl-Bosch-Strasse 38**
**D - 6700 Ludwigshafen (DE)**

(72) Erfinder: **Eicken, Karl, Dr.**
**Waldstrasse 53d**
**D - 6706 Wachenheim (DE)**
**Wuerzer, Bruno, Dr. Dipl.-Landwirt.**
**Wilhelm-Busch-Strasse 55**
**D - 6703 Limburgerhof (DE)**
**Rohr, Wolfgang, Dr.**
**Gontardstrasse 4**
**D - 6800 Mannheim 1 (DE)**

**EP 0 001 751 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# N-(Imdazolylmethyl)-acetanilide, ihre Verwendung als Herbizide und Verfahren zur Bekämpfung unerwünschten Pflanzenwuchses

Die vorliegende Erfindung betrifft Acetanilide, Herbizide, welche diese Verbindungen enthalten, sowie Verfahren zur Bekämpfung unerwünschten Pflanzenwuchses mit diesen Verbindungen.

Halogenacetanilide mit herbiziden oder wachstumsregulierenden Eigenschaften sind bekannt. Eine beachtliche wirtschaftliche Bedeutung hat das N-Methoxymethyl-$\alpha$-chloracet-2,6-diäthylanilid (US-PS 3.547.620). Auch ein Wirkstoff, der eine 1,3-Dioxolan-2-yl-methylgruppe trägt, ist bekannt (DT-OS 2 405 510). Die Stärke bekannter Acetanilide liegt in ihrer herbiciden Wirkung gegen zahlreiche aus Samen hervorgehende Gräser. Von der oben zuerst genannten Verbindungen werden außerdem noch einige wenige dikotyle Arten bekämpfen. Es fehlen jedoch Wirkstoffe, welche neben der herbiciden Wirkung gegen Gräser auch eine verbesserte Wirkung gegen breitblättrige Arten haben und vor allem eine markante Steigerung der Verträglichkeit bei bislang von bekannten Wirkstoffen geschädigten Kulturpflanzen zeigen.

Es wurde gefunden, daß Acetanilide der Formel

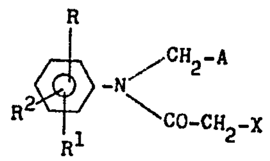

I

in der

R und $R^1$ für Alkyl mit bis zu 5 Kohlenstoffatomen steht,

$R^2$ für Wasserstoff,

X für Chlor steht und

A für ein über ein Ring-Stickstoffatom gebundenes Imidazol steht, das einfach oder mehrfach durch Halogen oder Alkyl oder Halogen und Alkyl mit jeweils bis zu 4 Kohlenstoffatomen substituiert sein kann, wobei A auch für ein Salz des Imidazols stehen kann,

bei guter herbizider Wirkung eine vorzügliche Selektivität bei wichtigen Kulturpflanzen aufweisen.

Aufgrund des Standes der Technik war es überraschend, daß sich gerade die erfindungsgemäßen imidazolsubstituierten Acetanilide durch dieses hohe Maß an Kulturpflanzenverträglichkeit auszeichnen.

Die neuen Wirkstoffe sind je nach Zielsetzung und Dosis geeignet zur selektiven Bekämpfung von unerwünschten Pflanzen in bestimmten Kulturpflanzenbeständen, bestehend aus krautigen oder verholzenden Pflanzenarten, zur Wachstumsregulierung durch Hemmung des Pflanzenwachstums oder bei entsprechenden hohen Aufwandmengen zur totalen Bekämpfung des Pflanzenwuchses.

Bei niedrigen Dosierungen besitzen eine besonders ausgeprägte Selektivität gegenüber Nutzpflanzen die erfindungsgemäßen Verbindungen entsprechend Formel I, bei denen R und R′ Substituenten in 2- und 6-Stellung am Phenylkern sind und jeweils Alkyl mit bis zu 5 Kohlenstoffatomen bedeuten.

Für die Symbole R, $R^1$, $R^2$, X und Y in der Formel I kommen in Betracht:

Für R und $R^1$ Alkyl bis zu 5 Kohlenstoffatomen wie Methyl, Äthyl, n-Propyl, i-Propyl, n-Butyl, sec.-Butyl, i-Butyl, tert.-Butyl, normale und verzweigte Pentylreste;

für X Chlor;

für A ein über ein Ring-Stickstoffatom gebundenes Imidazol, wie Imidazol, 2-Methylimidazol, 4(5)-Chlorimidazol, 2-Chlorimidazol, 4,5-Dichlorimidazol, Trichlorimidazol, 2-Methyl-4,5-dichlorimidazol, 2-Äthyl-4,5-dichlorimidazol, 2-Isopropyl-4,5-dichlorimidazol, 4(5)-Bromimidazol, 4,5-Dibromimidazol, Tribromimidazol, 2-Methyl-4,5-dibromimidazol, 2-Brom-4,5-Dichlorimidazol.

Darüber hinaus kann der Rest A auch salzartig an eine der üblichen starken anorganischen oder organischen Säuren, wie Chlorwasserstoffsäure, Bromwasserstoffsäure, Salpetersäure, Schwefelsäure, Tetrafluorborsäure, Fluorsulfonsäure, Ameisensäure, eine halogenierte Carbonsäure, z.B. Trichloressigsäure, eine Alkansulfonsäure, z.B. Methansulfonsäure, eine halogenierte Alkansulfonsäure, z.B. Trifluormethansulfonsäure, Perfluorhexansulfonsäure, eine Arylsulfonsäure, z.B. Dodecylbenzolsulfonsäure, gebunden sein.

Die neuen Acetanilide der Formel I können nach folgenden Verfahren hergestellt werden:

Die Acetanilide der Formel I werden durch Umsetzung von 2-Halogen-N-halogenmethyl-acetaniliden der Formel II mit einem Imidazol der Formel H—A nach folgender Reaktionsgleichung erhalten:

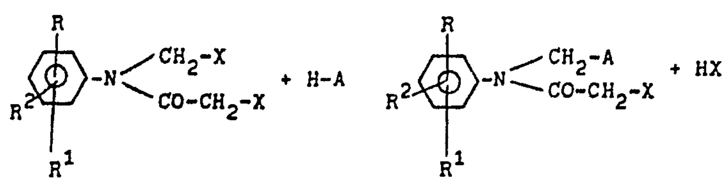

II I

Dabei haben die Substituenten R, $R^1$, $R^2$, A und X die oben genannte Bedeutung.

Einige der 2-Halogen-N-halogenmethyl-acetanilide der Formel II sind aus der DT-AS 15 42 950 bekannt; andere können auf analoge Weise durch Umsetzung der entsprechenden Azomethine mit einem Halogenacetylhalogenid hergestellt werden.

Das Imidazol wird zweckmäßig in mindestens äquimolarer Menge bezogen auf 2-Halogen-N-halogenmethyl-acetanilid, eingesetzt.

Der bei der Umsetzung freiwerdende Halogenwasserstoff wird zweckmäßigerweise durch geeignete Bindemittel, wie organischen Basen, beispielsweise tertiäre Amine oder anorganische Basen, beispielsweise Alkalimetall- oder Erdalkalimetallcarbonate bzw. -bicarbonate, abgefangen. Das Halogenwasserstoff-Bindemittel wird in mindestens äquimolarer Menge, bezogen auf eingesetztes Imidazol zugegeben.

Es ist zweckmäßig, die Reaktion in einem Lösungsmittel durchzuführen, das gegenüber 2-Halogen-N-halogenmethyl-acetaniliden inert ist. Hierzu eignen sich: Kohlenwasserstoffe, wie Petroläther, Ligroin, Cyclohexan, Toluol, Xylol; Äther wie Diäthyäther; Diisopropyläther, Dimethoxyäthan, Tetrahydrofuran, Dioxan, Anisol; halogenierte Kohlenwasserstoffe, wie Dichlormethan, Chloroform, 1,2-Dichloräthan, Tetrachlorkohlenstoff, Chlorbenzol; Ketone, wie Aceton, Methyläthylketon; Ester, wie Essigester, butylacetat, Sulfone, wie Dimethylsulfoxid, Tetrahydrothiophen-1,1-dioxid. Auch Gemische dieser Lösungsmittel können verwendet werden.

Die Reaktion kann bei Temperaturen ab 0°C aufwärts durchgeführt werden. Zur Beschleunigung der Umsetzung ist es vorteilhaft, beim Siedepunkt des Lösungsmittels bzw. des Lösungsmittelgemisches zu arbeiten, jedoch nicht über 200°C. Vorzugsweise arbeitet man bei Temperaturen im Bereich von 50 bis 150°C. Nach Ende der Reaktion wird abfiltriert, und das Produkt wird, gegebenenfalls nach vorheriger Waschung, aus dem Filtrat auf eine der herkömmlichen Weisen isoliert. Bei Verwendung eines mit Wasser mischbaren Lösungsmittels ist es meist vorteilhaft, dieses nach der Filtration durch Verdampfen zu entfernen und durch ein mit Wasser nicht mischbares Solvens zu ersetzen.

Weiterhin können die neuen Acetanilide der Formel I durch Umsetzung der 2-Halogen-N-halogenmethyl-acetanilide der Formel II mit einem Salz des Imidazols der Formel $M^{\oplus}A^{\ominus}$ nach folgender Reaktionsgleichung hergestellt werden:

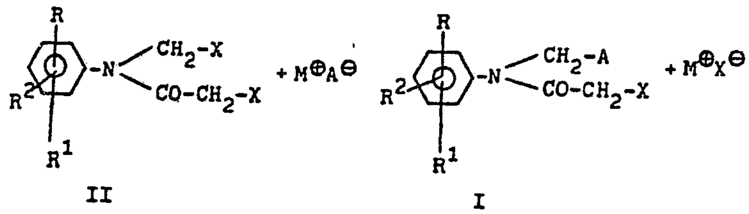

Dabei haben die Substituenten R, $R^1$, $R^2$, A und X die oben genannten Bedeutungen, $M^{\oplus}$ steht für das Silberion, ein Alkalimetallion oder ein Äquivalent Erdalkalimetallion.

Die Alkalimetall-, Erdalkalimetall- bzw. Silber-Imidazole $M^{\oplus}A^{\ominus}$ werden in bekannter Weise durch Umsetzung des Imidazols H—A mit Alkalimetallen oder starken Basen, wie Alkalimetallhydroxid, -alkoholat, -amid oder -hyrid oder Silberhydroxid, unter Freisetzung von Wasserstoff, Alkohol bzw. Ammoniak hergestellt.

Die Umsetzung der Salze $M^{\oplus}A^{\ominus}$ mit den 2-Halogen-N-halogenmethylacetaniliden der Formel II wird vorteilhafterweise in polaren, aprotischen Lösungsmitteln, wie Nitrilen, z.B. Acetonitril, wie Amiden, z.B. Dimethylformamid, wie Polyäthern, z.B. Diäthylenglykoldimethyläther, Triäthylenglykoldimethyläther, wie Sulfonen, z.B. Tetrahydrothiophen-1,1-dioxid, Sulfoxiden, z.B. Dimethylsulfoxid, Ketonen, wie z.B. Aceton, Methyläthylketon, Diisopropylketon, bei Temperaturen zwischen −30° bis +50°C, vorzugsweise bei Raumtemperatur, durchgeführt. Die Ausgangsstoffe werden dabei zweckmäßigerweise in äquimolarem Verhältnis eingesetzt. Reaktionsprodukte werden—gegebenenfalls nach Abtrennen der gebildeten anorganischen Salze $M^{\oplus}X^{\ominus}$ und gegebenenfalls nach Austausch des polaren, aprotischen Solvens durch ein mit Wasser nicht mischbares Lösungsmittel—in üblicherweise isoliert.

Die Salze der Acetanilide der Formel I können in an sich bekannter Weise aus den Acetaniliden der Formel I, die nach einem der vorher beschriebenen Verfahren hergestellt werden können, durch Zusatz von mindestens äquimolaren Mengen starker anorganischer oder organischer Säuren, wie Chlorwasserstoffsäure, Bromwasserstoffsulfonsäure, Ameisensäure, halogenierte Carbonsäuren, z.B. Trichloressigsäure, Alkansulfonsäure, z.B. Methansulfonsäure, halogenierte Alkansulfonsäure, z.B. Trifluormethansulfonsäure, Perfluorhexansulfonsäure, Arylsulfonsäure, z.B. Dodecylbenzolsulfonsäure, erhalten werden.

Das folgende Beispiel erläutert die Herstellung der neuen Acetanilide und ihrer Salze. In dem Beispiel verhalten sich Gewichtsteile zu Volumenteilen wie Kilogramm zu Liter.

Beispiel 1

30,2 Gewichsteile 2-Methyl-4,5-dichlorimidazol werden unter Zusatz von 60 Volumenteilen Methanol in 35,0 Gewichtsteilen 30%iger methanolischer Natriummethylatlösung aufgelöst und im Vakuum bei 30° zur Trockne eingeengt. Der kristalline Rückstand wird in 200 Volumenteilen trockenem Acetonitril suspendiert. Unter Rühren werden bei 10°C 49,2 Gewichtsteile 2-Chlor-2',6'-dimethyl-N-chlormethylacetanilid portionsweise eingetragen und 16 Stunden bei Raumtemperatur gerührt. Nach dem Verdampfen des Lösungsmittels im Vakuum wird der Rückstand zwischen Chloroform und Wasser verteilt. Aus der Chloroformphase isoliert man nach dreimaligem Waschen mit Wasser, Trocknen und Umkristallisieren aus Toluol 50,0 Gewichtsteile 2 - Chlor - 2',6' - dimethyl - N - (2 - methyl - 4,5 - dichlorimidazol - 1 - yl - methyl) - acetanilid vom Fp. 170°C (Verbindung Nr. 8).

Folgende Verbindungen erhält man in entsprechender Weise:

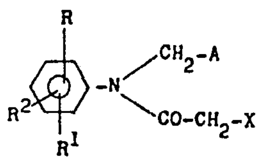

I

| Nr. | R | $R^1$ | $R^2$ | X | A | Fp. (°C) |
|---|---|---|---|---|---|---|
| 1 | $2-CH_3$ | $6-CH_3$ | H | Cl | 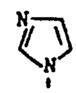 | 127 |
| 2 | $2-CH_3$ | $6-C_2H_5$ | H | Cl | ,, | 106 |
| 3 | $2-CH_3$ | $6-CH_3$ | H | Cl | 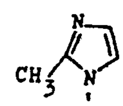 | 116 |
| 4 | $2-CH_3$ | $6-CH_3$ | H | Cl | 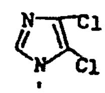 | 95 |
| 5 | $2-CH_3$ | $6-C_2H_5$ | H | Cl | ,, | 88 |
| 6 | $2-C_2H_5$ | $6-C_2H_5$ | H | Cl | ,, | 104 |
| 7 | $2-CH_3$ | $6-C_2H_5$ | H | Cl | 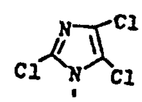 | 162 |
| 8 | $2-CH_3$ | $6-CH_3$ | H | Cl | 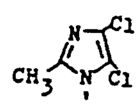 | 170 |
| 9 | $2-CH_3$ | $6-C_2H_5$ | H | Cl | ,, | 165 |
| 10 | $2-CH_3$ | $6-CH_3$ | H | Cl | 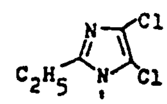 | 114 |
| 11 | $2-CH_3$ | $6-C_2H_5$ | H | Cl | 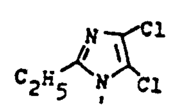 | 100 |

| Nr. | R | $R^1$ | $R^2$ | X | A | Fp. (°C) |
|---|---|---|---|---|---|---|
| 12 | 2–$CH_3$ | 6–$CH_3$ | H | Cl | 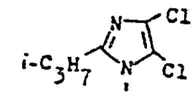 | 161 |
| 13 | 2–$CH_3$ | 6–$C_2H_5$ | H | Cl | ,, | 112 |
| 14 | 2–$CH_3$ | 6–$CH_3$ | H | Cl | 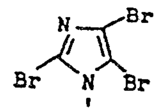 | 168 |
| 15 | 2–$CH_3$ | 6–$C_2H_5$ | H | Cl | ,, | 170 |
| 16 | 2–$CH_3$ | 6–$CH_3$ | H | Cl | 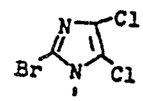 | 168 |
| 17 | 2–$CH_3$ | 6–$CH_3$ | H | Cl | 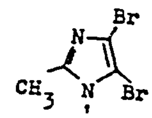 | 160 |

Die Erfindungsgemäßen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Oldispersionen, Pasten, Stäubemitteln, Streumitteln, Granulaten durch Versprühen, Vernebeln, Verstäuben, Verstreuen oder Gießen angewendet. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron, stark polare Lösungsmittel, wie Dimethylformamid, Dimethylsulfoxid, N-Methylpyrrilidon, Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht: Alkalimetall-, Erdalkalimetall-, Ammoniumsalze von Ligninsulfonsäure, Naphthalinsulfonsäure, Phenolsulfonsäure, Alkylarylsulfonate, Alkylsulfate, Alkylsulfonate, Alkalimetall- und Erdalkalimetall- salze der Dibutylnaphthalinsulfonsäure, Laurylläthersulfat, Fettalkoholsulfate fettsaure Alkalimetall- und Erdalkalimetallsalze, Salze sulfatierter Hexadecanole, Heptadecanole, Octadecanole, Salze von sulfatierten Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäure mit Phenol und Formaldehyd, Polyoxyäthylenoctylphenoläther, äthoxyliertes Isooctylphenol-, Octylphenol-, Nonylphenol, Alkylphenolpolyglykoläther, Tributylphenylpolyglykoläther, Alkalarylpolyätheralkohole, Isotridecylalkohol, Fettalkoholäthylenoxid Kondensate, äthoxyliertes Rizinusöl, Polyoxäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus,

Löß, Ton, Dolomit, Diatomeenerde, Calcium- und Magnesiumsulfat, Magnesiumoxid, gemahlene Kunststoffe, Düngemittel, wie Ammoniumsulfat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent. Die Aufwandmengen betragen 0,1 bis 10 kg Wirkstoff/ha.

Die neuen erfindungsgemäßen herbiziden Anilide können mit zahlreichen Vertretern anderer herbizider oder wachstumsregulierender Wirkstoffgruppen gemischt und gemeinsam ausgebracht werden. Beispielsweise kommen als Mischungskomponenten Diazine, Benzothiadiazinone, 2,6-Dinitroaniline, N-Phenyl-carbamate, Discarbamate, Thiolcarbamate, Halogencarbonsäuren, Triazine, Amide, Harnstoffe, Diphenyläther, Triazinone, Uracile und Benzofuranderivate in Betracht.

Außerdem ist es nützlich, die neuen erfindungsgemäßen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralstofflösungen, welche zur Behebung zon Ernährungs- oder Spurenelementmängeln eingesetzt werden.

Der Einfluß verschiedener Vertreter der erfindungsgemäßen Verbindungen auf das Wachstum von unerwünschten und erwünschten Pflanzen im Vergleich zu bekannten, chemisch ähnlichen Wirkstoffen wird in den folgenden Versuchen demonstriert. Die Versuchsserien werden im Gewächshaus durchgeführt.

Als Kulturgefäße dienten Plastikblumentöpfe mit 300 $cm^3$ Inhalt und lehmiger Sand mit etwa 1,5% Humus als Substrat. Die Samen der Testpflanzen entsprechend Tabelle 1 wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen und auch gleichzeitig die chemischen Mittel zu aktivieren. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Chemikalien beeinträchtigt wurde, und verhinderte das Verdampfen leicht flüchtiger Substanzen.

Die Aufstellung der Versuche erfolgte im Gewächshaus, wobei für wärmeliebende Arten wärmere Bereiche (25° bis 40°) und für solche gemäßigter Klimate 15 bis 30°C bevorzugt werden. Die Versuchsperiode erstreckte sich über 4 bis 6 Wochen. Während dieser Zeit wurden die Pflanzen gepflegt und ihre Reaktion auf die einzelnen Behandlungen ausgewertet. Die Tabelle 2 enthält die Prüfsubstanzen, die jeweiligen Dosierungen in kg/ha Aktivsubstanz und die Testpflanzenarten. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Sproßteile.

Ergebnis

Tabelle 2 enthält einige Zahlenwerte zur biologischen Charakterisierung der neuen Verbindungen. Man erkennt daraus vor allem die deutlich bessere Verträglichkeit für gewisse Kulturpflanzen im Gegensatz zu den bekannten Wirkstoffen.

Die Tabelle enthält Resultate aus Vorauflaufbehandlungen. Natürlich können die neuen Wirkstoffe auch zur Nachauflaufbehandlung eingesetzt werden. Hierbei erreicht man je nach Dosis der Wirkstoffe ein vollständiges Absterben oder bei geringeren Aufwandmengen lediglich eine wuchshemmende Wirkung.

TABELLE 1

Liste der Testpflanzen

| Botanischer Name | Deutsche Bezeichnung | Englische Bezeichnung |
|---|---|---|
| Alopacurus myosuroides | Ackerfuchsschwanz | slender foxtail |
| Beta vulgaris | Zuckerrübe | sugarbeet |
| Echinochloa crus galli | Hühnerhirse | bamyardgrass |
| Glycine max | Sojabohne | soybean |
| Gossypium hirsutum | Baumwolle | cotton |
| Setaria spp. | Borstenhirseart | foxtail spp. |
| Sorghum bicolor | Mohrenhirse | Sorghum |
| Zea mays | Mais | Indian corn |

TABELLE 2

Selektive herbizide Wirkung bei Vorauflaufanwendung im Gewächshaus

Grundkörper des Moleküls 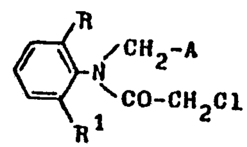

| Wirkstoff-Nr. | Substitutionen R | $R^1$ | A | kg/ha a.S. | Testpflanzen und % Schädigung: Beta vulgaris | Glycine max | Gossypium hirsutum | Sorghum bicolor | Zea mays | Alopecurus myosuroides | Echinochloa crus galli | Setaria spp. |
|---|---|---|---|---|---|---|---|---|---|---|---|---|
| 1 | | | | 1,0 | 0 | 0 | 0 | – | 0 | 20 | 40 | 20 |
| | | | | 2,0 | 0 | 0 | 0 | – | 0 | 50 | 50 | 25 |
| 4 | | | | 0,5 | 0 | 0 | 0 | 0 | 0 | 80 | 100 | 100 |
| | | | | 1,0 | 0 | 0 | 5 | 0 | 0 | 80 | 100 | 100 |
| | | | | 2,0 | 5 | 5 | 5 | 0 | 5 | 95 | 100 | 100 |
| 5 | | | | 0,5 | 0 | 0 | 10 | 0 | 0 | 100 | 100 | 100 |
| | | | | 1,0 | 0 | 0 | 10 | 0 | 0 | 100 | 100 | 100 |
| | | | | 2,0 | 5 | 0 | 20 | 0 | 5 | – | 100 | 100 |
| bekannt US–PS 3,547,620 | $C_2H_5$ | $C_2H_5$ | $-OCH_3$ | 2,0 | 60 | 0 | 40 | 80 | – | 72 | 100 | 100 |
| 11 | | | | 2,0 | 0 | 10 | 10 | – | 10 | 90 | 100 | – |
| 6 | | | | 2,0 | 10 | 10 | 10 | – | 10 | 70 | 100 | – |
| bekannt aus DT–0' 2,405,510 | $CH_3$ | $CH_3$ | 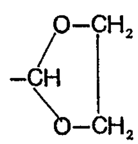 | 2,0 | 20 | – | 10 | 90 | 10 | 95 | 100 | 100 |

0 = keine Schädigung 100 = Pflanzen nicht aufgelaufen oder abgestorben

Beispiel 2

Man vermischt 90 Gewichtsteile der Verbindung 1 mit 10 Gewichtsteilen N-Methyl-$\alpha$-pyrrolidon und erhält eine Lösung, die zur Anwendung in Form kleinster Tropfen geeignet ist.

Beispiel 3

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel 4

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 5

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 6

20 Gewichtsteile des Wirkstoffs 2 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 7

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 8

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels besprüht wurde, innig vermischt. Man erhält auf diese Weise eine Aufbereitung des Wirkstoffs mit guter Haftfähigkeit.

Beispiel 9

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen 100 000 Gewichtsteilen Wasser erhält man eine wäßrige Dispersion, die 0,04 Gewichtsprozent Wirkstoff enthält.

Beispiel 10

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

## Patentansprüche

1. Acetanilide der Formel

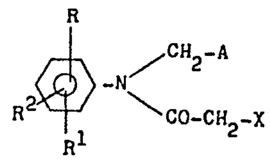

I

in der

R und $R^1$ für Alkyl mit bis zu 5 Kohlenstoffatomen steht,

$R^2$ für Wasserstoff,

X für Chlor steht und

A für ein über ein Ring-Stickstoffatom gebundenes Imidazol steht, das einfach oder mehrfach durch Halogen oder Alkyl oder Halogen und Alkyl mit bis zu 4 Kohlenstoffatomen substituiert sein kann, wobei A auch für Salze des Imidazols stehen kann.

2. 2 - Chlor - 2',6' - dimethyl - N - (4,5 - dichlorimidazol - 1 - yl - methyl) - acetanilid.

3. 2 - Chlor - 2' - methyl - 6' - äthyl - N - (4,5 - dichlorimidazol - 1 - yl - methyl) - acetanilid.

4. 2 - Chlor - 2',6' - diäthyl - N - (4,5 - dichlorimidazol - 1 - yl - methyl) - acetanilid.

5. 2 - Chlor - 2' - methyl - 6' - äthyl - N - (2 - äthyl - 4,5 - dichlorimidazol - 1 - yl - methyl) - acetanilid.

6. Herbizid, entahltend als Wirkstoff ein Acetanilid gemäß Anspruch 1.

7. Herbizid nach Anspruch 6, wobei das Acetanilid ausgewählt ist aus der Gruppe bestehend aus

2 - Chor - 2',6' - dimethyl - N - (4,5 -dichlorimidazol - 1 - yl - methyl) - acetanilid.

2 - Chlor - 2' - methyl - 6' - äthyl - N - (4,5 - dichlorimidazol - 1 - yl - methyl) - acetanilid.

2 - Chlor - 2',6' - diäthyl - N - (4,5 - dichlorimidazol - 1 - yl - methyl) - acetanilid.

2 - Chlor - 2' - methyl - 6' - äthyl - N - (2 - äthyl - 4,5 - dichlorimidazol - 1 - yl - methyl) - acetanilid.

8. Verfahren zur Bekämpfung unerwünschten Pflanzenwuchses, dadurch gekennzeichnet, daß man den Boden oder die Pflanzen behandelt mit einem Acetanilid gemäß Anspruch 1.

## Revendications

1. Acétanilides de formule

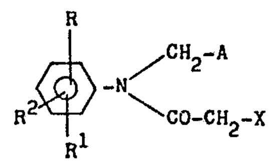

I

dans laquelle:

R et $R^1$ représentent un reste alkyle pouvant avoir jusqu'à 5 atomes de carbone,

$R^2$ est de l'hydrogène,

X est du chlore, et

A est un imidazol lié par un atome d'azote du noyau, qui peut être substitué une ou plusieurs fois par un halogène ou un reste alkyle ou un halogène et un reste alkyle pouvant avoir jusqu'à 4 atomes de carbone, A pouvant aussi représenter des sels de l'imidazol.

2. 2 - Chloro - 2',6' - diméthyl - N - (4,5 - dichlorimidazol - 1 - yl - méthyl) - acétanilide.

3. 2 - Chloro - 2' - méthyl - 6' - éthyl - N - (4,5 - dichlorimidazol - 1 - yl - méthyl) - acétanilide.

4. 2 - Chloro - 2',6' - diéthyl - N - (4,5 - dichlorimidazol - 1 - yl - méthyl) - acétanilide.

5. 2 - Chloro - 2' - méthyl - 6' - éthyl - N - (2 - éthyl - 4,5 - dichlorimidazol - 1 - yl - méthyl) - acétanilide.

6. Herbicide contenant comme principe actif un acétanilide selon la revendication 1.

7. Herbicide selon la revendication 6, dans lequel l'acétanilide est choisi dans le groupe constitué par:

le 2 - chloro - 2',6' - diméthyl - N - (4,5 - dichlorimidazol - 1 - yl - méthyl) - acétanilide,

le 2 - chloro - 2' - méthyl - 6' - éthyl - N - (4,5 - dichlorimidazol - 1 - yl - méthyl) - acétanilide,

le 2 - chloro - 2',6' - diéthyl - N - (4,5 - dichlorimidazol - 1 - yl - méthyl) - acétanilide,

le 2 - chloro - 2' - méthyl - 6' - éthyl - N - (2 - éthyl - 4,5 - dichlorimidazol - 1 - yl - méthyl)-acétanilide.

8. Procédé pour lutter contre la croissance des plantes adventices, caractérisé par la fait qu'on traite le sol ou les plantes avec un acétanilide selon la revendication 1.

## Claims

1. Acetanilides of the formula

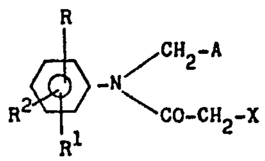

I

where R and $R^1$ each denotes alkyl of a maximum of 5 carbon atoms, $R^2$ denotes hydrogen, X denotes chlorine, and A denotes imidazole, or a salt thereof, which is attached via a ring nitrogen atom and may be mono- or polysubstituted by halogen or alkyl radicals or halogen and alkyl radicals each of a maximum of 4 carbon atoms.

2. 2 - Chloro - 2′,6′ - dimethyl - N - (4,5 - dichloroimidazol - 1 - yl - methyl) - acetanilide.

3. 2 - Chloro - 2′ - methyl - 6′ - ethyl - N - (4,5 - dichloroimidazol - 1 - yl - methyl) - acetanilide.

4. 2 - Chloro - 2′,6′ - diethyl - N - (4,5 - dichloroimidazol - 1 - yl - methyl) - acetanilide.

5. 2 - Chloro - 2′ - methyl - 6′ - ethyl - N - (2 - ethyl - 4,5 - dichloroimidazol - 1 - yl - methyl)-acetanilide.

6. A herbicide containing as active ingredient an acetanilide as claimed in claim 1.

7. A herbicide as claimed in claim 6 wherein the acetanilide has been selected from the group consisting of

2 - chloro - 2′,6′ - dimethyl - N - (4,5 - dichloroimidazol - 1 - yl - methyl) - acetanilide,

2 - chloro - 2′ - methyl - 6′ - ethyl - N - (4,5 - dichloroimidazol - 1 - yl - methyl) - acetanilide,

2 - chloro - 2′,6′ - diethyl - N - (4,5 - dichloroimidazol - 1 - yl - methyl) - acetanilide, and

2 - chloro - 2′ - methyl - 6′ - ethyl - N - (2 - ethyl) - 4,5 - dichloroimidazol - 1 - yl - methyl)-acetanilide.

8. A process for controlling unwanted plant growth, *characterized in that* the soil or the plants are treated with an acetanilide as claimed in claim 1.